# EUROPÄISCHE PATENTANMELDUNG

(11) **EP 0 538 688 A1**
(43) Veröffentlichungstag der Anmeldung: **28.04.1993**
(21) Anmeldenummer: 92117259.9
(22) Anmeldetag: 09.10.1992
(51) Int. Cl.: C07D 307/24, A61K 31/34

(54) **Neue substituierte Tetrahydrofurane, Verfahren zu ihrer Herstellung und ihre Verwendung als Arzneimittel**

(30) Priorität: 22.10.1991 DE 4134758
(71) Anmelder: BAYER AG, 51368 Leverkusen (DE)
(72) Erfinder: Kunisch, Franz, Dr., W-5068 Odenthal-Glöbusch (DE); Mittendorf, Joachim, Dr., W-5600 Wuppertal 1 (DE); Plempel, Manfred, Dr., W-5657 Haan 1 (DE)

(57) **Zusammenfassung**

Die vorliegende Erfindung betrifft neue substituierte Tetrahydrofurane der allgemeinen Formel (I) 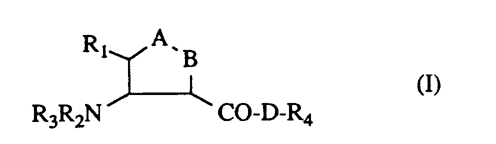
in welcher A, B, R₁, R₂, R₃, D und R₄ die in der Beschreibung angegebene Bedeutung haben, Verfahren zu ihrer Herstellung und ihre Verwendung als Arzneimittel, insbesondere als antimykotische Arzneimittel.

## Beschreibung

Die Erfindung betrifft neue substituierte Tetrahydrofurane, Verfahren zu ihrer Herstellung und ihre Verwendung als Arzneimittel, insbesondere als antimykotische Arzneimittel.

Es ist bereits bekannt, daß 2-Amino-3-homofuranoside eine antibakterielle Wirkung besitzen [vgl. JP 29 79 45(J6 3150-271-A)].

Außerdem werden in der Publikation Tetrahedron 26 (15), 3849 - 56 die Verbindungen 2-Amino-3-ethoxycarbonyl-dihydrofuran und 2-Amino-3-ethoxy-carbonyl-4-methyl-4,5-dihydrofuran in Form der konjugierten Säuren der entsprechenden Enaminoester beschrieben.

Die Erfindung betrifft nun neue substituierte Tetrahydrofurane der allgemeinen Formel (I), 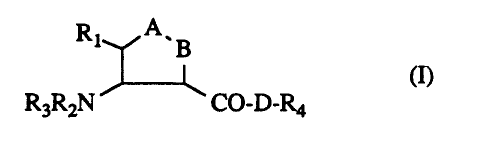
in welcher
- A und B: stets verschieden sind und für ein Sauerstoffatom oder für die Gruppe der Formel -CHR⁵ stehen,
worin
- R⁵: Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
- R¹: für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR⁶R⁷ substituiert ist,
worin
- R⁶ und R⁷: gleich oder verschieden sind und Wasserstoff, Phenyl oder geradkettiges oder verzweigtes Alkyl mit bis zu 6 Kohlenstoffatomen bedeuten,
- R²: für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel -SO₂R⁸ steht,
worin
- R⁸: geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano,Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR⁶R⁷ substitituiert sind,
worin
- R⁶ und R⁷: die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR⁶R⁷ oder -SO₂R⁸ substituiert ist,
worin
- R⁶, R⁷ und R⁸: die oben angegebene Bedeutung haben,
- R³: für Wasserstoff oder für geradkettiges oder verzweigtes Alkyl mit bis zu 8 Kohlenstoffatomen steht, das gegebenenfalls durch Phenyl substituiert ist,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauersoff- oder Schwefelatom oder für die 〉NH-Gruppe steht,
- R⁴: für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR⁶R⁷ oder -SO₂R⁸ substituiert sind,
worin
- R⁶, R⁷ und R⁸: die oben angegebene Bedeutung haben,
oder für den Fall, daß D für die 〉NH-Gruppe steht,
- R⁴: für die Gruppe der Formel -SO₂R⁸ steht,
worin
- R⁸: die oben angegebene Bedeutung hat,
gegebenenfalls in einer isomeren Form, und deren Säureadditions-Salze und Metallsalzkomplexe.

Die erfindungsgemäßen Verbindungen können auch in Form ihrer Salze vorliegen. Im allgemeinen seien hier Salze mit organischen oder anorganischen Basen oder Säuren genannt.
Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxygruppe besitzen. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Bevorzugt werden Verbindungen der allgemeinen Formel (I), in welcher
- A und B: stets verschieden sind und
für ein Sauerstoffatom oder für die Gruppe der Formel -CHR⁵ stehen,
worin
- R⁵: Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
- R¹: für Wasserstoff oder für geradkettiges oder verzweigtes Alkyl mit bis zu 6 Kohlenstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ substituiert ist,
worin
- R⁶ und R⁷: gleich oder verschieden sind und Wasserstoff oder geradkettiges oder verzweigtes Alkyl mit bis zu 4 Kohlenstoffatomen bedeuten,
- R²: für Wasserstoff oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder für Benzoyl steht, das gegebenenfalls wie oben beschrieben substituiert ist,
oder
für eine Gruppe der Formel -SO₂R⁸ steht,
worin
- R⁸: geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel -NR⁶R⁷ substituiert ist,
worin
- R⁶ und R⁷: die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel -NR⁶R⁷ oder -SO₂R⁸ substituiert ist,
worin
- R⁶, R⁷ und R⁸: die oben angegebene Bedeutung haben,
- R³: für Wasserstoff oder für geradkettiges oder verzweigtes Alkyl mit bis zu 6 Kohlenstoffatomen oder Benzyl steht,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauerstoff- oder Schwefelatom oder für die 〉NH-Gruppe steht,
- R⁴: für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ oder -SO₂R⁸ substituiert sind,
worin
- R⁶, R⁷ und R⁸: die oben angegebene Bedeutung haben,
oder für den Fall, daß D für die 〉NH-Gruppe steht,
- R⁴: für die Gruppe der Formel -SO₂R⁸ steht,
worin
- R⁸: die oben angegebene Bedeutung hat,
gegebenenfalls in einer isomeren Form und deren Säureadditions-Salze und Metallsalzkomplexe.

Besonders bevorzugt werden Verbindungen der allgemeinen Formel (I),
in welcher
- A und B: stets verschieden sind und
für ein Sauerstoffatom oder für die Gruppe der Formel -CHR⁵ stehen,
worin
- R⁵: Wasserstoff oder geradkettiges oder verzweigtes Alkyl mit bis zu 4 Kohlenstoffatomen bedeutet,
- R¹: für Wasserstoff oder
für geradkettiges oder verzweigtes Alkyl mit bis 4 Kohlenstoffatomen steht,
- R²: für Wasserstoff oder
für geradkettiges oder verzweigtes Alkyl mit bis zu 4 Kohlenstoffatomen steht, oder
für geradkettiges oder verzweigtes Acyl mit bis zu 4 Kohlenstoffatomen oder für eine Gruppe der Formel -SO₂R⁸ steht,
worin
- R⁸: geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
- R³: für Wasserstoff oder
für geradkettiges oder verzweigtes Alkyl mit bis zu 4 Kohlenstoffatomen steht,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauerstoff- oder ein Schwefelatom oder für die 〉NH-Gruppe steht,
- R⁴: für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel -NR⁶R⁷ oder -SO₂R⁸ substituiert sind,
worin
- R⁶und R⁷: gleich oder verschieden sind und Wasserstoff, Methyl oder Ethyl bedeuten
und
- R⁸: die oben angegebene Bedeutung hat,
oder im Fall, daß D für die 〉NH-Gruppe steht,
- R⁴: für die Gruppe der Formel -SO₂R⁸ steht,
worin
- R⁸: die oben angegebene Bedeutung hat,
gegebenenfalls in einer isomeren Form, und deren Säureadditions-Salze und Metallsalzkomplexe.

Ganz besonders bevorzugt sind die Verbindungen der allgemeinen Formel (I), in welcher die beiden Substituenten -NR²R³ und -CO-D-R⁴ in der cis-Stellung vorliegen.

Außerdem wurde ein Verfahren zur Herstellung der erfindungsgemäßen Verbindungen der allgemeinen Formel (I) gefunden, dadurch gekennzeichnet, daß man

### [A] im Fall, daß A für die -CHR⁵-Gruppe und B für ein Sauerstoffatom steht,

Verbindungen der allgemeinen Formel (II) 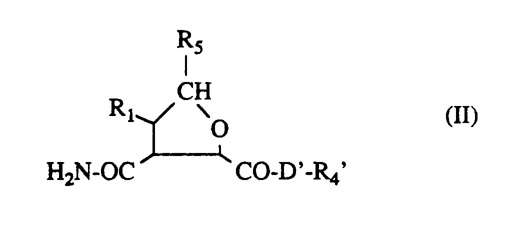
in welcher
- R¹ und R⁵: die oben angegebene Bedeutung haben,
- D': für ein Sauerstoffatom steht
und
- R^{4'}: für C₁-C₆-Alkyl steht,
zunächst in einer oxidativen Umlagerung mit Oxidationsmitteln, vorzugsweise Bleitetraacetat und Alkoholen (R⁹-OH) in Anwesenheit eines Katalysators, vorzugsweise Triethylamin,
in die Verbindungen der allgemeinen Formel (III) 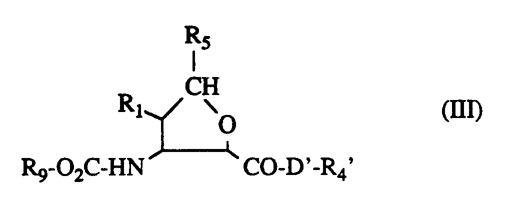
in welcher
- R¹, R^{4'}, R⁵ und D': die oben angegebene Bedeutung haben
und
- R⁹: für einen (C₁-C₆)-Alkylrest des entsprechenden Alkohols (R⁹-OH), vorzugsweise für tert.Butyl steht,
überführt,
und anschließend in inerten Lösemitteln, in Anwesenheit einer Säure, vorzugsweise Salzsäure, unter Abspaltung des Esterrestes (-CO₂R⁹) die entsprechenden Säureadditionssalze, vorzugsweise die Hydrochloride freisetzt,
oder

### [B] im Fall, daß A für ein Sauerstoffatom und B für die -CHR⁵-Gruppe steht,

Verbindungen der allgemeinen Formel (IV) 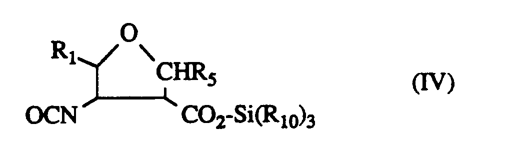
in welcher
- R¹ und R⁵: die oben angegebene Bedeutung haben
und
- R¹⁰: für einen C₁-C₃-Alkylrest steht,
in Ethern, vorzugsweise Diethylether, in Anwesenheit von Wasser
zunächst zu den Verbindungen der allgemeinen Formel (V) 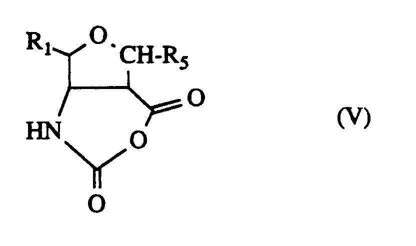
in welcher
- R¹ und R⁵: die oben angegebene Bedeutung haben,
umsetzt
und in einem nächsten Schritt mit Säuren, vorzugsweise Salzsäure und nachfolgend Propylenoxid, unter Ringöffnung in die Verbindungen der allgemeinen Formel (Ia) 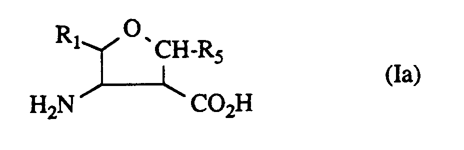
in welcher
- R¹ und R⁵: die oben angegebene Bedeutung haben,
überführt,
im Fall der Ester die Säuren nach üblicher Methode verestert,
im Fall der Säuren [(I)-> D = O; R⁴ = H] gegebenenfalls auch die entsprechenden Ester nach üblicher Methode verseift,
und im Fall der anderen für D und R⁴ oben aufgeführten Reste, ebenfalls nach üblichen Verfahren, wie beispielsweise Amidierung, Sulfonierng oder Sulfonamidierung, gegebenenfalls in Anwesenheit von Hilfsstoffen, wie Katalysatoren und Dehydratisierungsmitteln, ausgehend von den entsprechenden Carbonsäuren, gegebenenfalls unter vorgeschalteter Aktivierung, derivatisiert.

Die erfindungsgemäßen Verfahren können durch folgendes Formelschema beispielhaft erläutert werden:

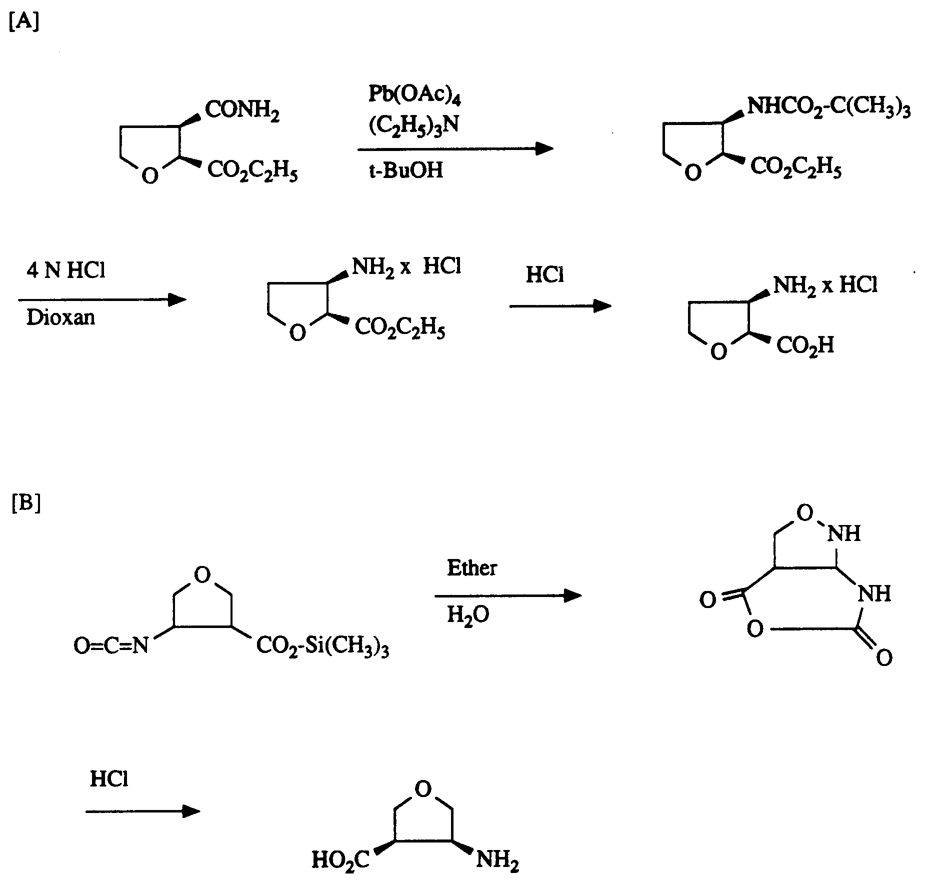

Als Lösemittel für Verfahren [A] und [B] kommen Wasser und alle inerten organischen Lösemittel in Frage, die sich unter den Reakionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind Ethanol, Tetrahydrofuran, Dioxan und Diethylether.

Die Reaktionstemperaturen können in einem größeren Breich varriiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Bei der Durchführung der erfindungsgemäßen Verfahrensvarianten [A] und [B] ist das Verhältnis der an der Reaktion beteiligten Stoffe beliebig. Im allgemeinen arbeitet man jedoch mit molaren Mengen der Reaktanden. Die Isolierung und Reinigung der erfindungsgmäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert In einigen Fällen kann es erforderlich sein, die erfindungsgemäßen Verbindungen durch Chromatographie zu reinigen.

Als Oxidationsmittel eignen sich beispielsweise Hypohalogenide wie [Bis-(trifluoracetoxy)-jod]benzol, (Hydroxy-p-toluolsulfonyloxyjodanyl)-benzol, Jodbenzol-diacetat, Jodosobenzol oder Bleitetraacetat. Bevorzugt ist Bleitetraacetat.

Als Katalysatoren für die oxidative Umlagerung eignen sich Basen wie organische Amine, Zinn(IV)chlorid oder Di-n-butylzinn-dilaureat, bevorzugt organische Amine. Die Reaktion verläuft auch ohne Katalysator.

Als Basen eignen sich organische Amine (Trialkyl(C1-C6)amine wie beispielsweise Triethylamin oder Heterocyclen wie Pyridin, Methylpiperidin, Piperidin oder Morpholin. Bevorzugt ist Triethylamin.

Als Säuren werden im allgemeinen Mineralsäuren eingesetzt. Bevorzugt werden hierbei Chlorwasserstoffsäure, Bromwasserstoffsäure, Schwefelsäure, Phosphorsäure oder aber Gemische der genannten Säuren eingesetzt. Bevorzugt ist Chlorwasserstoffsäure.

Die Katalysatoren werden im allgemeinen in einer Menge von 0 mol bis 10 mol, bevorzugt von 1,5 mol bis 3,5 mol bezogen auf 1 mol der Verbindungen der allgemeinen Formel (II) eingesetzt.

Die Säure wird im allgemeinen in einer Menge von 2 mol bis 30 mol, bevorzugt von 5 mol bis 15 mol jeweils bezogen auf 1 mol der Verbindungen der allgemeinen Formeln (III) und (V) eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Die Verseifung der Carbonsäureester kann ebenso mit einer der oben aufgeführten Säuren durchgeführt werden.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium- tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Beispielhaft für die oben aufgeführten Derivatisierungsmöglichkeiten sollen hier die Amidierung und Sulfonierung bzw. Sulfonamidierung erläutert werden.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Frerman et al., J. Biol. Chem. 225, 507 (1982) und N.B. Benoton, K. Kluroda, Int. Pept. Prot. Res. 13, 403 (1979), 17, 187 (1981)].

Die Sulfonierung oder Sulfoamidierung erfolgt in den oben erwähnten inerten Lösemitteln, gegebenenfalls unter Einsatz der ebenfalls oben aufgeführten Basen und Dehydratisierungsmittel.

Die Sulfonierung und Sulfoamidierung wird im allgemeinen bei Normaldruck durchgeführt. Es ist aber auch möglich, die Verfahren bei Unterdruck oder Überdruck durchzuführen (z.B. in einem Bereich von 0,5 bis 5 bar).

Die Sulfonierung und die Sulfoamidierung wird im allgemeinen in einem Temperaturbereich von 0°C bis +150°C, bevorzugt von +25°C bis +40°C, durchgeführt.

Zur Amidierung eignen sich im allgemeinen die literaturbekannten, käuflichen Amine und deren Derivate [vgl. Houben-Weyl, "Methoden der organischen Chemie", Bd .XI/1 und XI/2].

Die Sulfonierung und Sulfoamidierung erfolgen im allgemeinen ebenfalls mit den üblichen Sulfonsäuren und deren aktivierte Derivate [vgl. Houben-Weyl, "Methoden der organischen Chemie", Band IX, S 407 ff; Beilstein 11, 26].

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator bezogen auf 1 mol Reaktionspartner ein.

Sowohl die Veresterung als auch die Amidierung können gegebenenfalls über aktivierte Stufen der Carbonsäuren, wie beispielsweise Säurehalogenide, die aus der entsprechenden Säure durch Umsetzung mit Thionylchlorid, Phosphortrichlorid, Phosphorpentachlorid, Phosphortribromid oder Oxalylchlorid hergestellt werden können, verlaufen.

Die Säureadditions-Salze der Verbindungen der Formel (I) können in einfacher Weise nach üblichen Salzbildungsmethoden, z.B. durch Lösen einer Verbindung der Formel (I) in einem geeigneten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Verbindungen der allgemeinen Formel (II) sind größenteils neu, insbesondere die entsprechenden cis-Isomere, und können hergestellt werden, indem man
Verbindungen der allgemeinen Formel (VI) 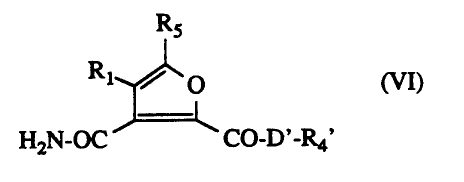
in welcher
- R¹, R^{4'}, R⁵ und D': die oben angegebene Bedeutung haben,
nach literaturbekannter Methode, vorzugsweise durch katalystische Hydrierung in Anwesenheit von Ruthenium reduziert [vgl. Polym. J. 7, 72 (1975)].

Die Verbindungen der allgemeinen Formel (VI) sind an sich bekannt oder können nach üblicher Methode hergestellt werden [vgl. JACS, 77, 1055,4069].

Die Verbindungen der allgemeinen Formel (III) sind neu und können nach dem oben aufgeführten Verfahren hergestellt werden.

Die Verbindungen der allgemeinen Formel (IV) sind neu und können hergestellt werden, indem man
Verbindungen der allgemeinen Formel (VII) 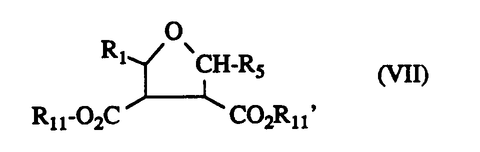
in welcher
- R¹ und R⁵: die oben angegebene Bedeutung haben
und
- R¹¹ und R^{11'}: gleich oder verschieden sind und für einen C₁-C₄-Alkylrest stehen,
zunächst mit Alkalihydroxiden, wie Natrium-, Kalium- oder Lithiumhydroxid, vorzugsweise Lithiumhydroxid, in einem Lösemittelgemisch, vorzugsweise Tetrahydrofuran und Wasser, zu den entsprechenden Carbonsäuren verseift, anschließend diese mit Propionsäureanhydrid in einem Temperaturbereich von +80°C bis +180°C, vorzugsweise bei 150°C zu den Verbindungen der allgemeinen Formel (VIII) 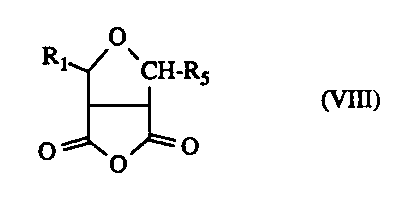
in welcher
- R¹ und R⁵: die oben angegebene Bedeutung haben,
umsetzt
und in einem letzten Schritt diese mit Verbindungen der allgemeinen Formel (IX)

(R¹⁰)₃SiN₃ (IX)

in welcher
- R¹⁰: die oben angegebene Bedeutung hat,
in einem Temperaturbereich von +20°C bis +80°C, vorzugsweise bei 80°C,
umsetzt.

Die Verbindungen der allgemeinen Formel (VII) sind teilweise bekannt [vgl. EP 22 086; J. Chem. Soc., Perkin Trans u2 (6), 875 - 886; Org. Coat. Plast. Chem. 44, 108-114; J. Macromol. Sci. Chem. A 13 (4), 477 -501] oder neu und können dann nach dem oben aufgeführten Verfahren hergestellt werden.

Die Verbindungen der allgemeinen Formel (VIII) sind größtenteils neu und können beispielsweise nach dem oben aufgeführten Verfahren hergestellt werden.

Die Verbindungen der allgemeinen Formel (IX) sind an sich bekannt oder können nach üblicher Methode hergestellt werden [vgl. z.B. Fieser 1, 1236; 3, 316; 5, 719; 6, 632; 7, 394; 9, 21 oder 10, 14].

Die Verbindungen der allgemeinen Formel (V) und (Ia) sind ebenfalls neu und können nach dem oben aufgeführten Verfahren hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Verbindungen der allgemeinen Formel (I) und ihre Säureadditions-Salze weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden: Alle Dermatomykosen und Systemmykosen, insbesondere solche, die durch die oben genannten Erreger hervorgerufen werden.

Die erfindungsgemäßen Verbindungen wurden in den Applikationsarten i.v., s.c. und orale Gabe am Modell der Mäuse-Candidose auf ihre antimykotische in vivo-Wirksamkeit geprüft:
Männliche CF₁-SPF-Mäuse wurden mit 1-3 x 10⁶ Sprosszellen von C. albicans pro Tier durch Injektion der Keimsuspension in phys. NaCl-Lösung (0,2 ml/Tier) in die Schwanzvene infiziert. Nicht behandelte Kontrolltiere entwickeln unter diesen Infektionsbedingungen eine Nierencandidose und sterben zu 95-100% der eingesetzten Tiere innerhalb 6 Tagen p.i. an dieser Infektion. Wurden infizierte Tiere täglich 2 mal, beginnend mit dem Tag der Infektion, mit den erfindungsgemäßen Verbindungen oral oder parenteral in Dosen 2 x 25 bis 2 x 50 mg/kg Körpergewicht über 2 - 5 Tage behandelt, so überlebten 60-90% der Tiere die Infektion in gutem Zustand. Die C.albicans-Keimzahlen in den Nieren der infizierten und behandelten Tiere liegen am 4. Tag p.i. durchschnittlich um 2 Zehnerpotenzen unter denen von unbehandelten, infizierten Kontrolltieren.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/der Trägerstoffen, gegebenenfalls unter Verwendung von Emulgiermittlen und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

### Ausgangsverbindungen

### Beispiel I

### 2,3-cis-tetrahydrofuran-3-carbonsäureamid-2-carbonsäureethylester

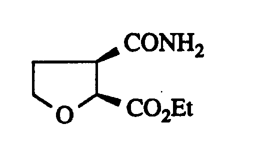

Eine Lösung von 2,3-cis-Furan-3-carbonsäureamid-2-carbonsäureethylester (1,00 g, 523 mmol) in 60 ml Ethanol wird in Gegenwart von 500 mg Rhodium auf Aktivkohle (5%) bei 120°C und 80 bar hydriert. Man filtriert, wäscht mit Ethanol und zieht das Solvens im Vakuum ab. Der Rückstand wird an Kieselgel chromatographiert (Essigester: Methanol = 10:1)
Ausbeute: 0,73 g (75% der Theorie)
C₈H₁₃NO₄ (187,2)
Smp.: 88°C

### Beispiel II

### 2,3-cis-3-(tert.Butyloxycarbonyl)amino-tetrahydro-2-furan-carbonsäureethylester

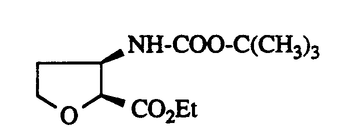

Eine Lösung der Verbindung aus Beispiel I (0,45 g, 2,40 mmol) in 20 ml tert.Butanol wird mit Blei(IV)acetat (1,15 g, 2,60 mmol) versetzt und 15 min unter Rückfluß erhitzt. Triethylamin (1 ml) wird zugetropft und die Mischung wird weitere 2 h unter Rückfluß erhitzt. Man zieht das Lösemittel im Vakuum ab, versetzt mit 30 ml Ether, filtriert und zieht den Ether im Vakuum ab. Der Rückstand wird an Kieselgel chromatographiert (Essigester: Petrolether = 1:3).
Ausbeute: 0,208g (33% der Theorie)
C₁₂H₂₁NO₅ (259,3)
Smp.: 75°C

### Beispiel III

### Tetrahydrofuran-3,4-dicarbonsäure

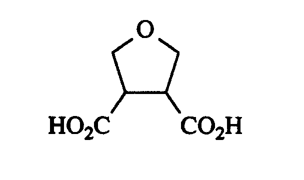

40 g (0,21 mol) 3,4-Dicarboxymethyl-tetrahydrofuran werden mit 10,4 g (0,43 mol) Lithiumhydroxid in einem Lösemittelgemisch aus 150 ml Tetrahydrofuran und 80 ml H₂O 20 h bei Raumtemperatur gerührt. Tetrahydrofuran wird weitgehend abdestilliert und der wäßrige Rückstand mit 6 N Schwefelsäure auf pH 1 eingestellt. Nach Sättigen mit NaCl extrahiert man mit Essigester, vereinigt die organischen Phasen und trocknet über Magnesiumsulfat. Die filtrierte Lösung wird eingeengt; es werden 23,4g (70% der Theorie) eines hellen Öls erhalten.
¹H-NMR (200 MHz, DMSO): δ = 3,28 (m, 2H); 3,75 - 4,00 (m, 4H).

### Beispiel IV

### (±)-cis-Tetrahydrofuran-3,4-carbonsäureanhydrid

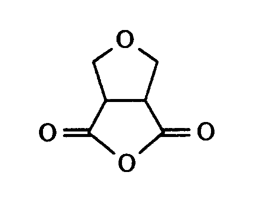

23 g (0,14 mol) (±)-3,4-Dicarboxy-tetrahydrofuran werden zusammen mit 100 ml Propionsäureanhydrid 6,5 h bei 150°C erhitzt. Nach Destillation werden 11 g (54% der Theorie) der Zielverbindung erhalten.
IR (CH₂Cl₂-Lsg.): 1790 und 1845 (breit) cm⁻¹

### Beispiel V

### (±)-cis-2-Isocyanato-3-carboxytrimethylsilyl-tetrahydrofuran

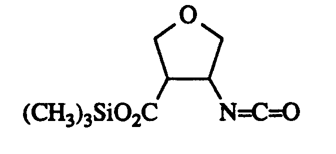

12 g (0.084 mol) (±)-cis-Tetrahydrofuran-3,4-dicarbonsäureanhydrid werden zusammen mit 13 ml (0.098 mol) Azidotrimethylsilan langsam auf 80°C erwärmt. Nach 45 min Rühren bei 80°C läßt man abkühlen und engt im Vakuum ein; es werden 17 g (88% der Theorie) der Zielverbindung erhalten, die ohne weitere Reinigung weiter umgesetzt werden kann.
IR (CH₂Cl₂-Lsg.): 1710,1790,2260 cm⁻¹.

### Beispiel VI

### (±)-cis-2-Amino-tetrahydrofuran-3-N-carbonsäureanhydrid

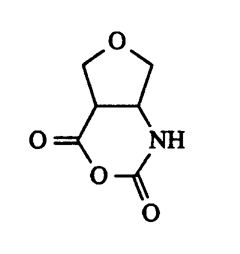

15 g (0,065 mol) (±)-cis-2-Isocyanato-3-carboxytrimethylsilyl(tetrahydrofuran) werden in 100 ml Diethylether mit 0,5 ml Wasser 5 min kräftig gerührt und die Lösung dann über Nacht bei +4°C auskristallisieren gelassen. Man erhält so 5,5 g (53% der Theorie) der Zielverbindung mit dem Smp von 133 - 139°C.
MS: m/e (% rel. Int.) 158 (MH⁺) (1); 127 (65); 55 (100).

### Herstellungsbeispiele

### Beispiel 1

### 2,3-cis-3-Amino-tetrahydro-2-furan-carbonsäureethylester Hydrochlorid

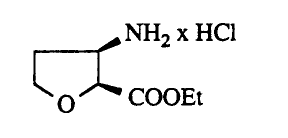

Eine Lösung der Verbindung aus Beispiel II (0,150 g, 0,58 mmol) in 1,5 ml einer 4 N Lösung von HCl in Dioxan wird 2 h bei Raumtemperatur gerührt Die Lösung wird im Vakuum eingeengt und der Rückstand 2 h bei 60°C/0,1 Torr getrocknet.
Ausbeute: 0,105 g (93% der Theorie)
C₇H₁₃NO₃ x HCl (159,2 x 36,5)
Smp.: 153°C

### Beispiel 2

### 2,3-cis-4-Amino-tetrahydro-2-furancarbonsäure Hydrochlorid

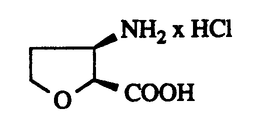

Eine Lösung der Verbindung aus Beispiel 1 (0,054 g / 0,28 mmol) in 5 ml 3 N Salzsäure wird 2 h unter Rückfluß erhitzt. Die Lösung wird im Vakuum eingeengt und der Rückstand 2 h bei 50°C /0,1 Torr. getrocknet.
Ausbeute: 0,045 g (98% der Theorie)
C₅H₉NO₃ x HCl (131,1 x 36,5)
Smp.: 214°C

### Beispiel 3

### (±)-cis-2-Amino-3-carboxy-tetrahydrofuran

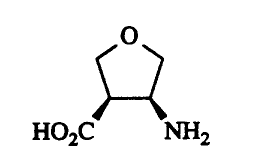

Zu 10 ml 6 N HCl werden 0,8 g (5 mmol) (±)-cis-2-Amino-tetrahydrofuran-3-N-carbonsäureanhydrid gegeben und 40 min bei Raumtemperatur gerührt. Man engt zur Trockne ein, nimmt den öligen Rückstand in 5 ml Ethanol auf und rührt mit 0,8 ml Propylenoxid 12 h bei Raumtemperatur. Nach Abfiltrieren und Trocknen im Hochvakuum erhält man 0,4g (60% der Theorie) mit dem Smp. 215 - 217°C.
¹H-NMR (200 MHz, D₂O): δ = 3,42 (m, -CHCO₂H; 1H)
MS (FAB): 132 (MH⁺)

## Patentansprüche

1. Substituierte Tetrahydrofurane der allgemeinen Formel (I), 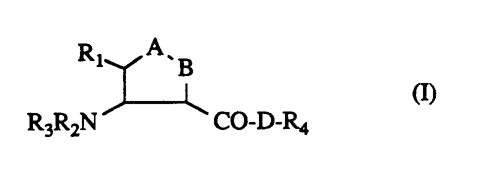 in welcher
A und B stets verschieden sind und
für ein Sauerstoffatom oder für die Gruppe der Formel -CHR⁵ stehen,
worin
R⁵ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
R¹ für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxy-carbonyl mit jeweils bis zu 6 Kohlenstoffatomen oder durch eine Gruppe der Formel -NR⁶R⁷ substituiert ist,
worin
R⁶ und R⁷ gleich oder verschieden sind und Wasserstoff, Phenyl oder geradkettiges oder verzweigtes Alkyl mit bis zu 6 Kohlenstoffatomen bedeuten,
R² für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen, oder
für Benzoyl steht, das gegebenenfalls wie oben beschrieben substituiert ist, oder
für eine Gruppe der Formel -SO₂R⁸ steht,
worin
R⁸ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR⁶R⁷ substitituiert sind,
worin
R⁶ und R⁷ die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR⁶R⁷ oder -SO₂R⁸ substituiert ist,
worin
R⁶, R⁷ und R⁸ die oben angegebene Bedeutung haben,
R³ für Wasserstoff oder für geradkettiges oder verzweigtes Alkyl mit bis zu 8 Kohlenstoffatomen steht, das gegebenenfalls durch Phenyl substituiert ist,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder Schwefelatom oder für die 〉NH-Gruppe steht,
R⁴ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR⁶R⁷ oder -SO₂R⁸ substituiert sind,
worin
R⁶, R⁷ und R⁸ die oben angegebene Bedeutung haben,
oder für den Fall, daß D für die 〉NH-Gruppe steht,
R⁴ für die Gruppe der Formel -SO₂R⁸ steht,
worin
R⁸ die oben angegebene Bedeutung hat.

2. Substituierte Tetrahydrofurane der allgemeinen Formel (I) gemäß Anspruch 1, in welcher
A und B stets verschieden sind und
für ein Sauerstoffatom oder für die Gruppe der Formel -CHR⁵ stehen,
worin
R⁵ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
R¹ für Wasserstoff oder für geradkettiges oder verzweigtes Alkyl mit bis zu 6 Kohlenstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ substituiert ist,
worin
R⁶ und R⁷ gleich oder verschieden sind und Wasserstoff oder geradkettiges oder verzweigtes Alkyl mit bis zu 4 Kohlenstoffatomen bedeuten,
R² für Wasserstoff oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Aryl mit bis zu 6 Kohlenstoffatomen oder für Benzoyl steht, das gegebenenfalls wie oben beschrieben substituiert ist, oder
für eine Gruppe der Formel -SO₂R⁸ steht,
worin
R⁸ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel -NR⁶R⁷ substituiert ist,
worin
R⁶ und R⁷ die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel -NR⁶R⁷ oder -SO₂R⁸ substituiert ist,
worin
R⁶, R⁷ und R⁸ die oben angegebene Bedeutung haben,
R³ für Wasserstoff oder für geradkettiges oder verzweigtes Alkyl mit bis zu 6 Kohlenstoffatomen oder Benzyl steht,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder Schwefelatom oder für die 〉NH-Gruppe steht,
R⁴ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ oder -SO₂R⁸ substituiert sind,
worin
R⁶, R⁷ und R⁸ die oben angegebene Bedeutung haben,
oder für den Fall, daß D für die 〉NH-Gruppe steht,
R⁴ für die Gruppe der Formel -SO₂R⁸ steht,
worin
R⁸ die oben angegebene Bedeutung hat

3. Substituierte Tetrahydrofurane der allgemeinen Formel (I) gemäß Anspruch 1, in welcher
A und B stets verschieden sind und
für ein Sauerstoffatom oder für die Gruppe der Formel -CHR⁵ stehen,
worin
R⁵ Wasserstoff oder geradkettiges oder verzweigtes Alkyl mit bis zu 4 Kohlenstoffatomen bedeutet,
R¹ für Wasserstoff oder
für geradkettiges oder verzweigtes Alkyl mit bis zu 4 Kohlenstoffatomen steht,
R² für Wasserstoff oder
für geradkettiges oder verzweigtes Alkyl mit bis zu 4 Kohlenstoffatomen steht, oder
für geradkettiges oder verzweigtes Acyl mit bis zu 4 Kohlenstoffatomen oder
für eine Gruppe der Formel -SO₂R⁸ steht,
worin
R⁸ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
R³ für Wasserstoff oder
für geradkettiges oder verzweigtes Alkyl mit bis zu 4 Kohlenstoffatomen steht,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder ein Schwefelatom oder für die 〉NH-Gruppe steht,
R⁴ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel -NR⁶R⁷ oder -SO₂R⁸ substituiert sind,
worin
R⁶und R⁷ gleich oder verschieden sind und Wasserstoff, Methyl oder Ethyl bedeuten
und
R⁸ die oben angegebene Bedeutung hat,
oder im Fall, daß D für die 〉NH-Gruppe steht,
R⁴ für die Gruppe der Formel -SO₂R⁸ steht,
worin
R⁸ die oben angegebene Bedeutung hat.

4. Verfahren zur Herstellung der Verbindungen der allgemeinen Formel (I) gemäß Anspruch 1, dadurch gekennzeichnet, daß man
[A] im Fall, daß A für die -CHR⁵-Gruppe und B für ein Sauerstoffatom steht,
Verbindungen der allgemeinen Formel (II) 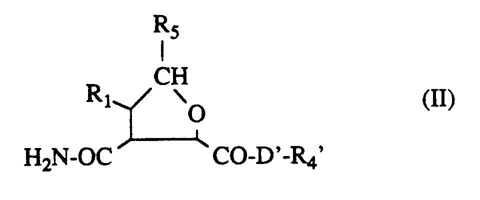 in welcher
R¹ und R⁵ die oben angegebene Bedeutung haben,
D' für ein Sauerstoffatom steht
und
R^{4'} für C₁-C₆-Alkyl steht,
zunächst in einer oxdativen Umlagerung mit Oxidationsmitteln, vorzugsweise Bleitetraacetat und Alkoholen (R⁹-OH) in Anwesenheit eines Katalysators, vorzugsweise Triethylamin,
in die Verbindungen der allgemeinen Formel (III) 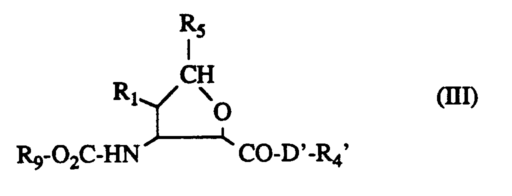 in welcher
R¹, R^{4'}, R⁵ und D' die oben angegebene Bedeutung haben
und
R⁹ für einen (C₁-C₆)-Alkylrest des entsprechenden Alkohols (R⁹-OH), vorzugsweise für tert.Butyl steht,
überführt,
und anschließend in inerten Lösemitteln, in Anwesenheit einer Säure, vorzugsweise Salzsäure, unter Abspaltung des Esterrestes (-CO₂R⁹) die entsprechenden Säureadditionssalze, vorzugsweise die Hydrochloride freisetzt,
oder
[B] im Fall, daß A für ein Sauerstoffatom und B für die -CHR⁵-Gruppe steht,
Verbindungen der allgemeinen Formel (IV) 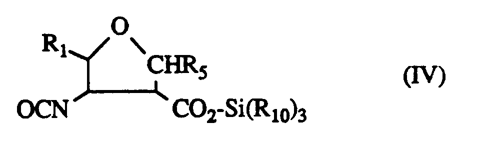 in welcher
R¹ und R⁵ die oben angegebene Bedeutung haben
und
R¹⁰ für einen C₁-C₃-Alkylrest steht,
in Ethern, vorzugsweise Diethylether, in Anwesenheit von Wasser
zunächst zu den Verbindungen der allgemeinen Formel (V) 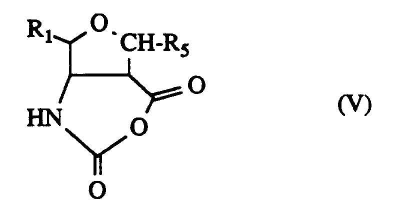 in welcher
R¹ und R⁵ die oben angegebene Bedeutung haben,
umsetzt
und in einem nächsten Schritt mit Säuren, vorzugsweise Salzsäure und nachfolgend Propylenoxid, unter Ringöffnung in die Verbindungen der allgemeinen Formel (Ia) 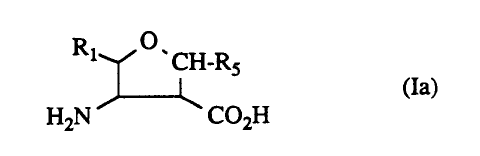 in welcher
R¹ und R⁵ die oben angegebene Bedeutung haben,
überführt,
im Fall der Ester die Säuren nach üblicher Methode verestert,
im Fall der Säuren [(I)-> D = O; R⁴ = H] gegebenenfalls auch die entsprechenden Ester nach üblicher Methode verseift,
und im Fall der anderen für D und R⁴ oben aufgeführten Reste, ebenfalls nach üblichen Verfahren, wie beispielsweise Amidierung, Sulfonierung oder Sulfonamidierung, gegebenenfalls in Anwesenheit von Hilfsstoffen, wie Katalysatoren und Dehydratisierungsmitteln, ausgehend von den entsprechenden Carbonsäuren, gegebenenfalls unter vorgeschalteter Aktivierung, derivatisiert.

5. Verwendung der Verbindung der allgemeinen Formel (I) gemäß Anspruch 1 zur Bekämpfung von Krankheiten.

6. Arzneimittel enthaltend Verbindungen der allgemeinen Formel (I) gemäß Anspruch 1.

7. Verwendung von Verbindungen der allgemeinen Formel (I) gemäß Anspruch 1 zur Herstellung von Arzneimittel für die Human- oder Veterinärmedizin.
